# EUROPEAN PATENT SPECIFICATION

(11) **EP 2 651 968 B1**
(45) Date of publication and mention of the grant of the patent: **15.10.2014**
(21) Application number: 11799166.1
(22) Date of filing: 19.12.2011
(51) Int. Cl.: D01D 5/12, D01F 4/02, C07K 14/435, D01D 5/00

(54) **PARALYSATION OF SILKWORM LARVAE**
PARALYSIEREN VON SEIDENWURM-LARVEN
PARALYSATION DES LARVES DE VERS A SOIE

(30) Priority: 17.12.2010 GB 201021451
(43) Date of publication of application: 23.10.2013
(73) Proprietor: Nefila Ltd., Beckely, Oxford OX3 9TF (GB)
(72) Inventor: VOLLRATH, Fritz, Beckely, Oxford OX3 9TF (GB); WOODS, Alexander, St. Aldates, Oxford OX1 1 DW (GB)
(74) Representative: Harrison, Robert John
(86) International application number: PCT/EP2011/073189
(87) International publication number: WO 2012/080510

(56) References cited:
- WO-A1-2005/049899
- SOON-DUCK HA ET AL: "Isolation and structure determination of a paralytic peptide from the hemolymph of the silkworm, Bombyx mori", PEPTIDES, vol. 20, no. 5, 3 August 1999 (1999-08-03) , pages 561-568, XP55021078, DOI: 10.1016/S0196-9781(99)00008-X
- M KAMIMURA ET AL: "Molecular Cloning of Silkworm Paralytic Peptide and Its Developmental Regulation", BIOCHEMICAL AND BIOPHYSICAL RESEARCH COMMUNICATIONS, vol. 286, no. 1, 1 August 2001 (2001-08-01), pages 67-73, XP55021080, ISSN: 0006-291X, DOI: 10.1006/bbrc.2001.5365

## Description

### FIELD OF THE INVENTION

The field of the present disclosure relates to a method and use for immobilising silk-producing Lepidopteran larvae and a method, device and use for forced silking from an immobilised silk-producing Lepidopteran larva.

### BACKGROUND OF THE INVENTION

The order Lepidoptera belongs to the class of insects. Lepidoptera is one of the most speciose orders in the world, encompassing moths and the three superfamilies of butterflies, skipper butterflies, and moth-butterflies. Many species of the order Lepidoptera are of economic interest by virtue of their important natural role through pollination or the silk they produce, for example *Bombyx mori.* The term "Lepidoptera" in this disclosure is used for the order itself and "Lepidopterans" for the members of the order Lepidoptera.

Species of Lepidopterans undergo a form of metamorphism called complete metamorphism. The life cycle of Lepidopterans normally consist of an egg, larva, pupa, and an imago or adult. Lepidopterans usually reproduce sexually and are oviparous (egg-laying), though some species give to live birth in a process called ovoviviparity. The larvae, or first stage in their life cycle after hatching, look very different from the adults and come in a variety of shapes and sizes. The larvae develop rapidly with several generations in a year, however some species may take up to 3 years to develop. The different instars, or moults, are regulated by hormones like prothoracicotropic hormone which in turn stimulates the production of moulting hormones (ecdysone and its homologues) and the release of molting hormones into the hemolymph, telling the Lepidopteran to start molting.

After about 5 to 7 instars the larva puparium, a sclerotized or hardened cuticle of the last larval instar, develops into the pupa. The pupa may be covered in silk and attached with many different types of debris or nothing at all depending on the species. The pupa is enclosed in a cocoon. The time it takes for pupae to emerge will vary between species. The adult will emerge from the cocoon either by using abdominal hooks or a projection from the head or enzymes that dissolve silk components.

The term "silkworm" is used in this disclosure to describe silk-producing larvae of bombicid, saturnid, lasiocampid and thaumatopeoid species. The term "mulberry silkworm" is used in this disclosure for the larvae of the Lepidopteran *Bombyx mori* and the term "wild silkworm" is used for the larvae of moths of the families bombycoidea (other than *B. mori*) saturniidae, lasiocampidae, mimallonidae, geometroidea, rhopalocera and noctuoidea that produce large larval cocoons.

The saturnid species of wild silkworms *Antheraea pernyi, Antheraea yamamai, Antheraea militta, Antheraea assama, Philosamia Cynthia ricini* and *Philosamia Cynthia pryeri* are currently used for the commercial silk production. Other saturnid as well as lasiocampid silkworms for example Gonometa spp. have great potential for the commercial production of silk.

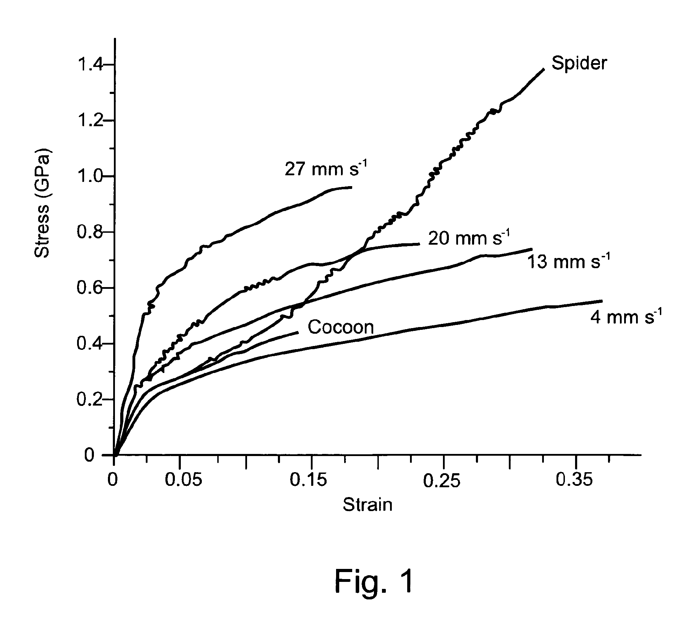

Silk is obtained commercially by direct reeling from a cocoon or less commonly by carding cocoons. However, it has been shown that artificial removal of silk (termed "forced silking") directly from immobilised 5th instar *Bombyx mori* silkworms under steady and controlled conditions in the laboratory is highly advantageous in that the forced silking produces fibres that are mechanically superior to naturally spun silk fibres (Fig. 1). Pérez-Rigueiro J., Elices M., Llorca J. and Viney C. have taught "Tensile properties of silkworm silk obtained by forced silking" (Journal of Applied Polymer Science 82(8): 1928-1935 (2001)). Similar disclosures have been made by Shao Z. and Vollrath, F. "Surprising strength of silkworm silk" (Nature 418: 741 (2002)); Khan Md.M.R., Morikawa H., Gotoh Y., Miura M., Ming Z., Sato Y. and Iwasa M. "Structural characteristics and properties of Bombyx mori silk fiber obtained by different artificial forcibly silking speeds" (International Journal of Biological Macromolecules 42(3): 264-270 (2008)) and Fu C., Porter D., Chen X., Vollrath F., Shao Z. "Understanding the Mechanical Properties of Antheraea Pernyi Silk-From Primary Structure to Condensed Structure of the Protei" (Advanced Functional Materials, 21 729-737 (2011)).

The methods disclosed by the above authors to immobilise the larvae all involve physical restraint and have the following limitations. The immobilisation by physical restraint requires considerable skill and time adding greatly to the cost of silk production and allow for only small lengths of silk fibre to be obtained. Furthermore the immobilisation produced by physical restraint is often incomplete. Even small and sporadic movements of some of the larvae or of muscles associated with the internal silk press can result in variation in draw rate and/or draw force leading to a lack of consistency in the mechanical properties of the silk. Finally, unless mandibles are painstakingly restrained, the larvae may use the mandibles to cut through the silk fibre reeled artificially from the spigot of the larvae causing costly delays in the production. These limitations mean that currently no silk fibre is produced commercially by the forced silking (direct reeling) from the larvae.

It has been disclosed by Ha SD, Nagata S, Suzuki A, Kataoka H. "Isolation and structure determination of a paralytic peptide from the hemolymph of the silkworm, Bombyx mori" (Peptides 20:561-8 (1999)) and Skinner W.S., Dennis, P.A., Li J.P., Summerfelt R.M., Carney, R.L., Quistad, G.B. "Isolation and identification of paralytic peptide from hemolymph of the lepidopteran insects Manduca sexta, Spodoptera exigua, and Heliothis virescens" (J. Biol. Chem. 266:12,873-12,877 (1991)) that rapid and rigid paralysis of the 5th instar mulberry silkworms can be produced by sub-cuticular injection of hemolymph drawn from the 5th instar mulberry larvae that has stopped feeding prior to spinning. The hemolymph derived from a donor silkworm must be exposed to air for ten minutes prior to the sub-cuticular injection. The active principle, *Bombyx mori* paralytic protein (bmPP), responsible for the paralysing effect has been isolated as disclosed by Ha SD, Nagata S, Suzuki A, Kataoka H. "Isolation and structure determination of a paralytic peptide from the hemolymph of the silkworm, Bombyx mori" (Peptides 20:561-8 (1999)) and cloned and sequenced as disclosed by Kamimura M., Nakahara Y., Kanamori Y., Tsuzuki S., Hayakawa Y., Kiuchi M. in their paper "Molecular cloning of silkworm paralytic peptide and its developmental regulation." published in Biochem. Biophys. Res. Commun. 286:67-73(2001). Kamimura et al. also disclosed that the expression of the bmPP was upregulated by juvenile hormone and 20-hydroxyecdysone in the 5^{th} instar silkworm thus providing an explanation of how the secretion of the bmPP is timed in the last instar. The bmPP is expressed in a hematopoietic organ-wing imaginal disc complex, suggesting that the bmPP is involved in both immune response and the hematopoietic process.

A wide range of synthetic or natural insecticides produce paralysis in silkworms. For example, A. M. Heimpel and T. A. Angus in their paper "Bacterial insecticides" published in 1960 as contribution Number 17, Insect Pathology Research Institute. Insect Pathology Research Institute, Sault Ste. Marie, Ontario, Canada http://mmbr.asm.org/cgi/reprint/24/3/266.pdf (downloaded on 18.01.2010) disclosed that bacterial products derived from a number of different infections of the mulberry silkworms can cause rapid paralysis of the silkworm larvae. However a risk of human morbidity is associated with the use of the synthetic pesticides. Deliberately raising and distributing the bacterial products capable of paralysing and killing the silkworm larvae would be imprudent in silk-producing regions.

There is still considerable scope for improvements in methods for immobilising Lepidopteran larvae and for forced silking directly from the immobilised Lepidopteran larvae.

Transgenic modification of the silkworm has been well documented in the last decade. Yamao M., Katayama N., Nakazawa H., Yamakawa M., Hayashi Y., Hara S., Kamei K., and Mori, H. "Gene targeting in the silkworm by use of a baculovirus" (Genes and Development 13, 511-516 (1999)) and Tamura T, Thibert C, Royer C, Kanda T, Abraham E, Kamba M, Komoto N, Thomas JL, Mauchamp B, Chavancy G, Shirk P, Fraser M, Prudhomme JC, Couble P. "Germline transformation of the silkworm Bombyx mori L. using a piggyBac transposon-derived vector" (Nature Biotechnology 18(1):81-4 (2000)), both describe early examples of methods that have become largely conventional. A number of patents have also been granted that use transgenic expression systems to produce a desirable phenotypic effect, for example: United States Patent Applications 20070067855, 20070056051, 20090183269 and United States Patent Number 6,551,825. Other recent references that discuss *piggyBac* vectors and methods for generating transgenic insects using the *piggyBac* vectors include, e.g., Handler A, McCoombs SD, Fraser MJ and Saul SH "The Lepidopteran transposon vector, piggyBac, mediates germline transformation in Mediterranean fruit fly" (Proc Natl Acad Sci 95, 7520-7525 (1998)); Fraser, MJ (2001) "The TTAA-specific family of transposable elements" In: Insect transgenesis: Methods and Applications. A. A. James and A. H. Handler, eds. CRC Press, Orlando, Fla.; Lobo N, Li X, Fraser MJ, Jr. "Transposition of the piggyBac element in embryos of Drosophila melanogaster, Aedes aegypti and Trichoplusia ni" (Mol. Gen. Genetics 261, 803-810 (1999)); Grossman, G.L., C.S. Rafferty, M.J. Fraser & M.Q. Benedict "The piggyBac element is capable of precise excision and transposition in cells and embryos of the mosquito, Anopheles gambiae" (Insect. Biochem. Mol. Biol. 30: 909-914 (2000)); Lobo NF, Li X, Hua-Van A, Fraser MJ., Jr. "Mobility of the piggyBac transposon in embryos of the vectors of Dengue fever (Aedes albopictus) and La Crosse encephalitis (Ae. triseriatus)" (Mol Genet Genomics. 265:66-71 (2001)); Lorenzen MD, Berghammer AJ, Brown SJ, Denell RE, Klinger M, Beeman RW "piggyBac-mediated germ-line transformation in the beetle Tribolium castaneum" (Insect Molec. Biol. 12:433-440 (2003)); Hacker U, Nystedt S, Barmchi MP, Horn C, Wimmer E "A piggyBac-based insertional mutagenesis in the presence of stably integrated P elements in Drosophila" (Proc Natl Acad Sci USA. 100 7720-5 (2003)); Sumitani M, Yamamoto DS, Oishi K, Lee JM, Hatakeyama M. "Germ-line transformation of the sawfly, Athalia rosae (Hymenoptera: Symphyta), mediated by a piggyBac-derived vector" (Insect Biochem. Mol. Biol. 33:449-458 (2003)); Horn C, Offen N, Nystedt S, Haecker U, Wimmer EA. "piggyBac-based insertional mutagensis and enhancer detection as a tool for functional insect genomics. (Genetics. 163:647-61 (2003)); and Tomita M, Munetsuna H, Sato T, Adachi T, Hino R, Hayashi M, Shimizu K, Nakamura N, Tamura T, Yoshizato K. "Transgenic silkworms produce recombinant human type III procollagen in cocoons" (Nat Biotechnol. 21, 52-6 (2003)).

The term "insect" used in this disclosure includes any stage of development of an insect, including a one-celled germ line cell, an egg and sperm, an early embryo, a larva, including any of a first through a fifth instar larva, a pupa, or an adult insect. It will be evident to someone skilled in the art which insect stage is suitable for a particular purpose, such as for storage or transport of an insect to a different location, for maintaining lines, for further genetic crosses, or the like.

The term "expression control sequence" used in this disclosure refers to a polynucleotide sequence that regulates expression of a polypeptide coded for by a polynucleotide to which it is functionally linked.

The term "promoter" used in this disclosure refers to a DNA sequence, generally directly upstream to the coding sequence, required for basal and/or regulated transcription of a gene. In particular, a promoter has sufficient information to allow initiation of transcription, generally having a transcription initiation start site and a binding site for the polymerase complex.

The term "recombinant" nucleic acid used in this disclosure refers to a nucleic acid that encodes a paralysis-inducing polypeptide which is heterologous to the insect, and/or a nucleic acid which has been genetically engineered (e.g., cloned into a vector) before being introduced into the insect. The nucleic acid encoding a protein originating from a particular species of Lepidoptera (endogenous to that type of Lepidoptera), but engineered so as to be produced at increased levels, and then introduced back into that type of insect, is thus considered to be recombinant.

The term "homologues" used in this disclosure refers to genes or proteins having similar or identical biological functions. The similarity or identity of the biological functions can be reflected by sequence similarity or identity (at either the amino acid or nucleotide level) of about 50%, about 70% or about 90%. Sequence similarity or identity (at either the amino acid or nucleotide level) within defined regions of the molecule or across the full-length sequence can be determined through sequence alignments using computer software programs such as BLAST, ALIGN, DNAstar and INHERIT which employ various algorithms to measure homology. A person skilled in the art is familiar with these alignment programs. Sequence regions that are homologous may be called conserved, consensus or canonical sequences and represent the most common choice of base or amino acid at each position.

### BRIEF SUMMARY OF THE INVENTION

The present disclosure teaches a safe, convenient, efficient and cost-effective method of immobilising domesticated or wild silk-producing Lepidopteran larvae to allow for continuous forced silking from the larvae. The methods of immobilisation reduce the need for skilled handling of the silk-producing Lepidopteran larvae and are practicable on a mass-rearing scale. Silk with consistent and superior mechanical properties can be reeled directly from the immobilised silk-producing Lepidopteran larvae at a control and constant draw rate, draw tension and external draw down.

According to the present disclosure a method for immobilising silk-producing Lepidopteran larvae is provided comprising paralysing the silk-producing Lepidopteran larvae by at least one of transgenically expressing a polypeptide having paralytic effects in the silk-producing Lepidopteran larvae, injecting hemolymph, wherein the hemolymph is drawn from silk-producing Lepidopteran larvae and oxidised prior to the injecting, or injecting a natural or artificial polypeptide having paralytic effects. The polypeptide can be a protein.

The transgenically expressing may comprise at least one of introducing a viral vector, introducing a transposon-based vector, or regulating by an expression control sequence. The viral vector or the transposon-based vector may further comprise a marker gene. The expression control sequence may comprise at least one of inducible promoter or enhancer element for further controlling the transgenically expressing.

Furthermore the controlling of the transgenically expressing may comprise at least one of applying chemicals for inducing transgenic expression, applying temperature for inducing transgenic expression, or applying chemicals for repressing transgenic expression.

A further object of the present disclosure is that the polypeptide having paralytic effects is bmPP, a native homologue or a fragment thereof. The bmPP, the native homologue or the fragment thereof may be mutated but retaining the paralytic effects.

At least one of paralysis or activity of the polypeptide having paralytic effects may also be modified independently of transcriptional control signals or translational control signals.

A further object of the present disclosure is the use of the above-described methods for immobilising silk-producing Lepidopteran larvae.

Furthermore a method for forced silking from silk-producing Lepidopteran larvae is provided comprising immobilising the silk-producing Lepidopteran larvae and directly and forced silking from the silk-producing Lepidopteran larvae.

A device for silking from an immobilised silk-producing Lepidopteran larva is also provided, comprising a mechanical reeling mechanism, to which are attached the immobilised silk-producing Lepidopteran larvae, whereby the immobilised silk-producing Lepidopteran larvae are immobilised according to the above-described methods and a take up device for the reeled silk. A further object is the use of such a device, capable of working at a variety of speeds and onto a variety of reels either singly or multiply in parallel, for collecting silk. Such a device might contain environmental controllers that would allow controlling environmental conditions such as temperature and humidity.

A device for post-draw stretching of silk, comprising one or more sensors, one or more post-draw reels, an actuator and an actuator sensor, and a collecting reel, whereby the immobilised silk-producing Lepidopteran larvae are attached to the post-draw stretching device and the immobilised silk-producing Lepidopteran larvae are immobilised according to at least one of the above-described methods is also provided. The device may additionally comprise a wash bath through which silks are reeled. Such device might also contain temperature controllers that would allow controlled heating and cooling of the larva to affect the silk properties or to target specific environmental triggers for inducing paralytic expression, for example Notch.

### DESCRIPTION OF THE FIGURES

Figure 1 shows stress-strain curves of washed and degummed single-filament silkworm silk (motor-reeled at 25 °C at the indicated speeds), *Nephila* spider dragline silk (20 mm s⁻¹ at 25°C) and standard, degummed commercial silk from a cocoon spun by the animal in the natural 'figure of eight' at speeds oscillating between 4 and 15 mm s⁻¹ at 20 °C. The area under the stress-strain curve represents the energy that a fibre can take up before breaking, and thus indicates its toughness. Scale bar represents 10 µm. Immobilized silkworms (n = 4) were forcibly silked, each providing 3-6 single filaments, which were tested in a stretching rig (force resolution, 30 µN; time resolution, <5 ms for 1 mN; strain rate, 50% per min. [reproduced from Shao Z. and Vollrath, F. "Surprising strength of silkworm silk" Nature 418: 741 (2002)]

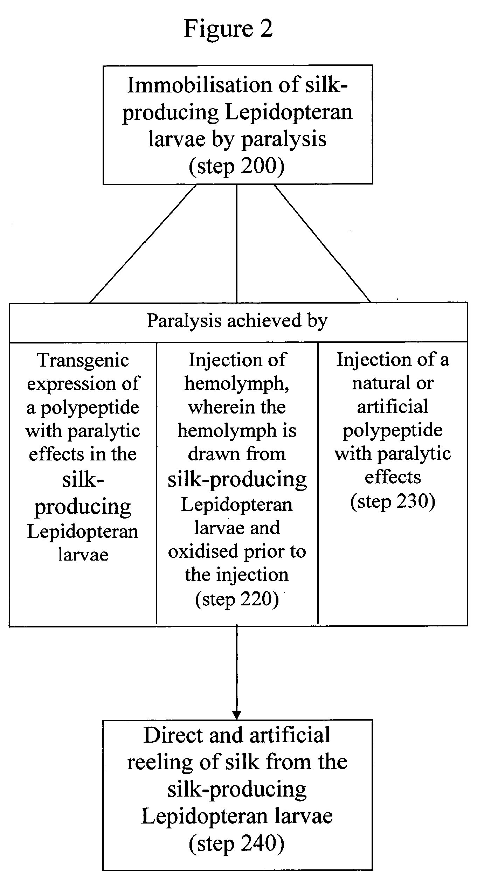

Figure 2 shows a flow chart involving the methodological steps of the present disclosure. The methodological steps involve immobilisation of silk-producing Lepidopteran larvae by paralysis (step 200), paralysis by at least one of transgenic expression of a polypeptide with paralytic effects in the silk-producing Lepidopteran larvae (step 210), injection of hemolymph, wherein the hemolymph is drawn from silk-producing Lepidopteran larvae and oxidised prior to the injection (step 220), or injection of a natural or artificial polypeptide with paralytic effects (step 230) and direct and artificial silking from the silk-producing Lepidopteran larvae (step 240).

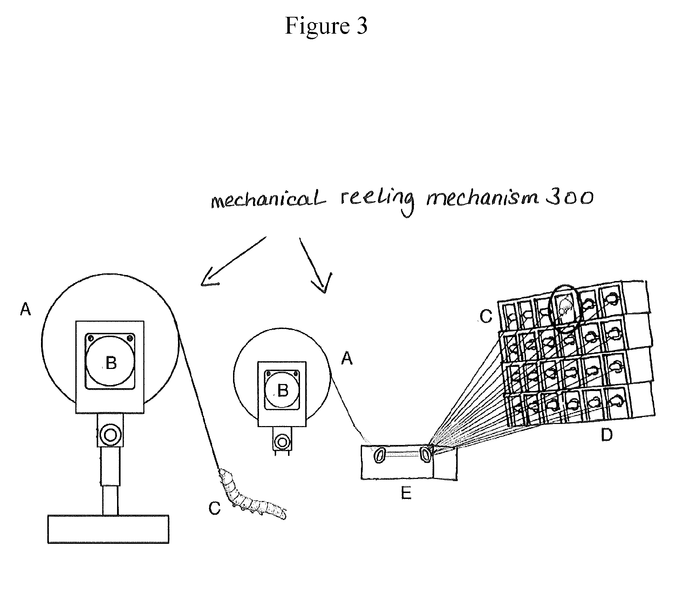

Figure 3 shows an example of a reeling device, a mechanical reeling mechanism 300, for forced silking. A are reels for reeling the silk, B are motors, C are silk-producing Lepidopteran larvae, D is a device to hold many spinning larvae and E is a wash bath through which silks are reeled.

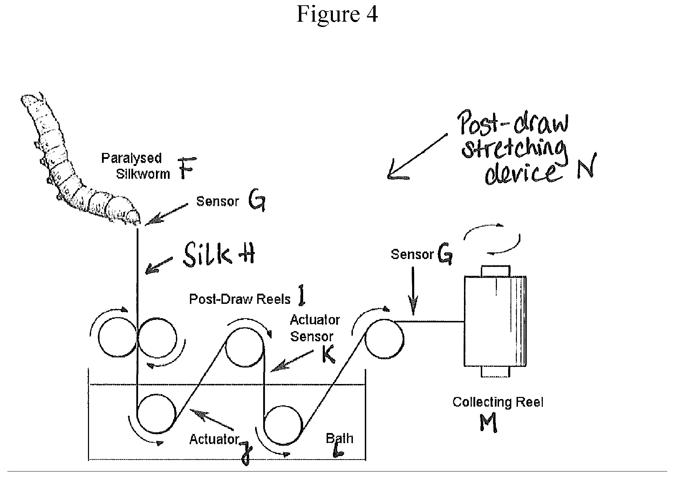

Figure 4 shows a post-draw stretching device N for reeled silk by way of example. F is a paralysed silkworm, G could be a sensor or set of sensors for silk dimensions and properties, for example filament motion and tension, and the sensor could also include thermistors for heating and cooling, H is the reeled silk, I are post-draw reels for reeling the silk, J is an actuator for stretching the silk, K is an actuator sensor for sensing the stretching of the silk, L is a wash bath through which silks are reeled, and M is a collecting reel.

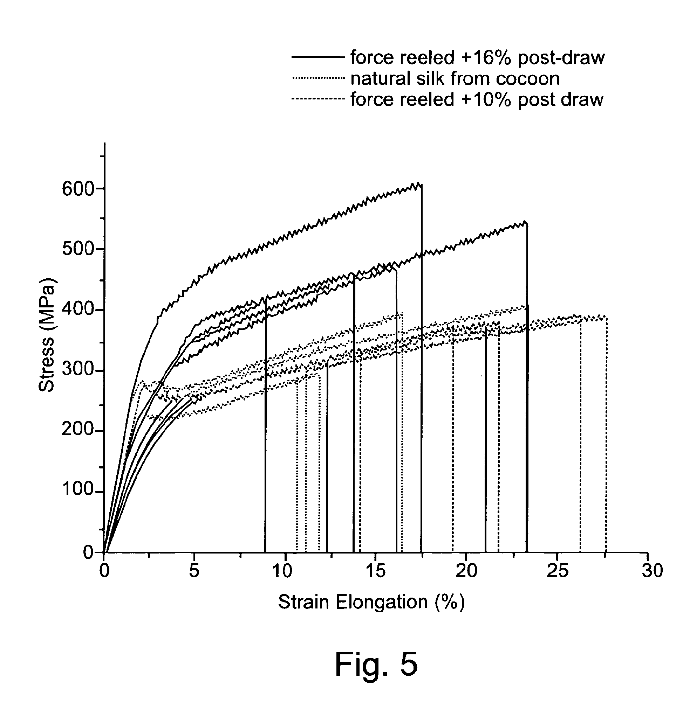

Figure 5 demonstrates the effect of small scale post-draw elongation/stretching by 10% and 16% on force reeled silk fibre in relation to silk reeled directly from a cocoon. The curves show stress as a function of strain elongation and the figure demonstrates the use of controlled paralysation to tune fibre properties and achieve predictability of properties.

### DETAILED DESCRIPTION OF THE INVENTION

The immobilization by paralysis of the silk-producing Lepidopteran larvae can be achieved in the following three methods which are illustrated in Fig. 2. The method for immobilizing silk-producing Lepidopteran larvae comprising paralyzing the silk-producing Lepidopteran larvae (step 200) by:
Method 1 (step 210): transgenically expressing a polypeptide having paralytic effects in the silk-producing Lepidopteran larvae.
Method 2 (step 220): injecting hemolymph, wherein the hemolymph is drawn from silk-producing Lepidopteran larvae and oxidized prior to the injecting, or
Method 3 (step 230): injecting a natural or artificial polypeptide having paralytic effects.

The first method (step 210) is achieved by producing transgenic larval silk-producing Lepidopterans in which some or all of the cells of a body of the transgenic larval silk-producing Lepidopterans contain a genetic construct that allows for expression of a native protein or polypeptide with paralytic effects in 5^{th} instar silk-producing Lepidopteran larvae. Such a native protein or polypeptide with paralytic effects includes, but is not limited to *Bombyx mori* paralytic peptide [bmPP] in *B. mori,* native homologues of this polypeptide in other wild silkworm or a fragment thereof. This method, once induced, can result in a direct synthesis of active protein or polypeptide causing the paralytic effect. Inactivated ones of the proteins or the polypeptides already native to the silk-producing Lepidopteran larvae can also be activated (for example, bmPP in hemolymph of *B. mori* larvae, or homologues of this polypeptide in other wild silkworm species of interest), causing the paralytic effect.

The second method (step 220) of the paralysis of the silk-producing Lepidopteran larvae is achieved by injecting hemolymph. The hemolymph is drawn from the silk-producing Lepidopteran larvae and oxidised prior to the injecting of the hemolymph. The Lepidopteran larvae from which the hemolymph is drawn include, for example, *B. mori* or other wild silkworms. The paralysed Lepidopteran larvae can be 5^{th} instar Lepidopteran larvae (for example *B. mori* or other wild silkworms) that have recently stopped accepting food. Thereby, a novel and non-obvious purpose for activating bmPP expression in fifth instar silk-producing Lepidopteran larvae is provided.

The third method (step 230) is achieved by isolating the native protein or polypeptide with paralytic effects from the hemolymph (for example *Bombyx mori* paralytic peptide [bmPP] from *B. mori,* native homologues of this polypeptide in other wild silkworm species of interest or a fragment thereof) extracted from Lepidopteran larvae or synthesised artificially, and injected into the 5^{th} instar silk-producing Lepidopteran larvae that have recently stopped accepting food. This reduces the quantity of skilled handling of silk-producing Lepidopterans needed to extract hemolymph, and is very cost-effective.

All three methods can be employed for immobilising the silk-producing Lepidopteran larvae in order to directly and artificially reel silk from the silk-producing Lepidopteran larvae (step 240). All three methods cause rapid onset of rigid paralysis and allow silk to be easily drawn from the immobilised silk-producing Lepidopteran larvae and attached to a mechanical reeling mechanism 300 capable of working at a variety of speeds, continuously and until the silk-producing Lepidopteran larvae has run out of silk (>300m) or cannot be manipulated to give more.

An illustrative example of this mechanical reeling mechanism 300 is shown in Fig. 3. The mechanical reeling mechanism 300 includes reels A for reeling the silk, B are motors, C are silk-producing Lepidopteran larvae, D is a device to hold many spinning ones of the silk-producing Lepidopteran larvae C and E is a wash bath through which silks are reeled. The mechanical reeling mechanism 300 allows the control of the time of spinning to optimise variables that have been shown to alter the mechanical properties of silks. Such variables include humidity (before and during silking) and air temperature (before and during spinning) as well as body temperature and body weight of the silk-producing Lepidopteran larvae C. We note that the silk after exciting the silk-producing Lepidopteran larva C can be diverted directly through one or a series of baths E to wash the silk off layers of undesirable sericin gum as well as to add layers of desirable compounds such as by way or example only protective or medically active coatings.

It will be appreciated that there could be more than one reel A operating at multiple speeds. It will be also appreciated that brins from the silk-producing Lepidopteran larva C can be twisted together at a controlled number of twists per unit length either directly linked to the reeling device or at a later stage.

Methods for generating transgenic silk-producing Lepidopterans are conventional. For example, in one embodiment, one or more genes to be introduced are cloned into a vector, such as a viral vector (e.g. an attenuated baculovirus vector, or a non-permissive viral vector that is not infective for the particular silk-producing Lepidopteran of interest). The sequences to be introduced into the silk-producing Lepidopteran are flanked by genomic sequences from the silk-producing Lepidopteran. The construct is then introduced into a silk-producing Lepidopteran egg (e.g. by microinjection), and the transgene(s) then integrate by homologous recombination of the flanking sequences into comparable sequences in the silk-producing Lepidopteran genome. One method according to the disclosure uses the techniques presented in Yamao M., Katayama N., Nakazawa H., Yamakawa M., Hayashi Y., Hara S., Kamei K., and Mori, H. "Gene targeting in the silkworm by use of a baculovirus" (Genes and Development 13, 511-516 (1999)). In their publication, *B. mori* were infected with a recombinant *AcMNPV* carrying a gene of interest flanked by sequences derived from the host genome. The virus successfully delivered its DNA, but failed to complete its infection cycle. The viral DNA recombined with the host genome via a homologous recombination event between the host sequences in the viral DNA and the same sequences in the *B. mori* genome.

In another embodiment, the vector is a transposon-based vector. One form of such transposon-based vectors is the viral vector (as disclosed above) that further comprises inverted terminal repeats of a suitable transposon, between which the transgene of interest is cloned. One or more transgenes of interest are cloned into the transposon-based vector. In some systems, the transposon-based vector carries its own transposase. However, generally, the transposon-based vector does not encode a suitable transposase. In this case, the vector is co-infected into a silk-producing Lepidopteran (e.g., a silk-producing Lepidopteran larva) with a helper virus or plasmid that provides the transposase. The recombinant vector and helper are introduced through conventional methods (such as microinjection) into an egg or early embryo; and the transgene(s) become integrated at a transposon site (for example sequences corresponding to the inverted terminal repeat of the transposon) in the silk-producing Lepidopteran genome. Suitable types of transposon-based vectors will be evident to the skilled worker. These include, e.g., *Minos, mariner, Hermes,* and *piggyBac.*

In an alternative embodiment, the transposon-based vector is a *"piggyBac"* vector. The TTAA-specific, short repeat elements are a group of transposons (Class II mobile elements) that have similar structures and movement properties. A typical *piggyBac* vector (formerly IFP2) is the most extensively studied of these insertion elements. *piggyBac* is 2.4 kb long and terminates in 13 bp perfect inverted repeats, with additional internal 19 bp inverted repeats located asymmetrically with respect to the ends [Cary L.C., Goebel M., Corsaro B.G., Wang H.G., Rosen E., Fraser M.J. "Transposon mutagenesis of baculoviruses: Analysis of Trichoplusia ni transposon IFP2 insertions within the FP-locus of nuclear polyhedrosis viruses" Virology 172, 156-69 (1989)]. The *piggyBac* vector may encode the transposase that allows its own movement; alternatively, the sequences coding for the transposase can be removed and the sequence coding for the transposase can be supplied via a helper plasmid or virus. The *piggyBac* vector has been deleted for non-essential genes, into which large inserts can be cloned. Inserts as large as about 15 kB can be cloned into certain ones of the *piggyBac* vectors. This allows, for example, for the insertion of about six or seven genes with their expression control sequences. Thus, a collection of paralytic peptides, marker proteins, or the like, can be introduced together via a single one of the transposon vector, into a single site in a silk-producing Lepidopteran genome.

Several ones of the *piggyBac* vectors have been developed for insect transgenesis. Two valuable "minimal constructs" for the movement of *piggyBac* vectored sequences were developed through analysis of deletion mutations both within and outside of the boundaries of the transposon [Li X, Lobo N, Bauser CA, Fraser MJ Jr. "The minimum internal and external sequence requirements for transposition of the eukaryotic transformation vector piggyback" Mol. Genet. Genomics. 266, 190-8 (2001)]. It is possible to increase the amount of genetic material mobilised by the *piggyBac* transposase by using constructs such as these minimal constructs and minimising the size of the vector. The minimal requirements for movement include the 5' and 3' terminal repeat domains and concomitant TTAA target sequences. Most of the internal domain may be removed, although some of this internal domain may be required for efficient translocation of the mobilised sequences into the genome of the insect. In addition, a minimum of 50 bases separating the TTAA target sites of the element is required for efficient mobilisation [Li X, Lobo N, Bauser CA, Fraser MJ Jr. "The minimum internal and external sequence requirements for transposition of the eukaryotic transformation vector piggyback" Mol. Genet. Genomics. 266, 190-8 (2001)].

The *piggBac* vector can transpose in insect cells while carrying a marker gene and movement of the *piggyBac* element can occur in cells from Lepidopteran species distantly related to the species from which it originated. For example, the *piggyBac* vector has been shown to transform various fruit fly species, *D. melanogaster, Anastrepha suspena, Bactrocera dorsalis,* as well as several mosquito species and five Lepidopteran species, *Pectinophora gossypiella, Trichoplusia ni, Cydia pomonella, Plutella xylostella* and *B. mori.*

Generally, a helper virus or plasmid that expresses a transposase is co-infected with the transposon-based vector as above. Expression of the transposase can be determined by the choice of promoter for the insect cistron or system (both terms being synonymously used) being tested. Suitable properties for promoters and examples are described elsewhere herein.

One method according to the disclosure could employ an approach suggested by Yamamoto M, Yamao M, Nishiyama H, Sugihara S, Nagaoka S, Tomita M, Yoshizato K, Tamura T, Mori H "New and highly efficient method for silkworm transgenesis using Autographa californica nucleopolyhedrovirus and piggyBac transposable elements" (Biotechnol Bioeng 88:849-853 (2004)). In this publication, a non-permissive host, *B. mori,* was infected with two recombinant *AcMNPVs.* One contained the *piggyBac* transposase under the regulation of *Drosophila* hsp70 promoter and the other encoded a green fluorescent protein to be inserted into the *B. mori* genome, flanked by the *piggyBac* inverted terminal repeats, under the control of the *B. mori* actin A3 promoter. The result was that the transposase expressed by one virus mobilised the DNA between the inverted terminal repeats in the other virus and integrated that DNA into the host silkworm genome.

The presence of resident copies of the *piggyBac* transposon in certain populations of *B. mori* does not appear to interfere with transposition of the transposon. Furthermore, strains of *B. mori* have been indentified which lack resident copies of the *piggyBac* transposon (for example, *Nistari*). *B. mori* embryos have been injected with *piggyBac* vectors, and transformants have been successfully recovered and characterised to confirm *piggyBac* mobilisation into the genome.

For further guidance on the use of baculovirus-based vectors, see, e.g., WO01/29204 and U.S. Pat. No. 6,551,825. Other recent references that discuss *piggyBac* vectors and methods for generating transgenic insects using them include, e.g., Handler A, McCoombs SD, Fraser MJ and Saul SH "The lepidopteran transposon vector, piggyBac, mediates germline transformation in Mediterranean fruit fly" (Proc Natl Acad Sci 95, 7520-7525 (1998)); Fraser, MJ (2001) "The TTAA-specific family of transposable elements" In: Insect transgenesis: Methods and Applications. A. A. James and A. H. Handler, eds. CRC Press, Orlando, Fla.; Lobo N, Li X, Fraser MJ, Jr. "Transposition of the piggyBac element in embryos of Drosophila melanogaster, Aedes aegypti and Trichoplusia ni" (Mol. Gen. Genetics 261, 803-810 (1999)); Grossman, G.L., C.S. Rafferty, M.J. Fraser & M.Q. Benedict "The piggyBac element is capable of precise excision and transposition in cells and embryos of the mosquito, Anopheles gambiae" (Insect. Biochem. Mol. Biol. 30: 909-914 (2000)); Lobo NF, Li X, Hua-Van A, Fraser MJ., Jr. "Mobility of the piggyBac transposon in embryos of the vectors of Dengue fever (Aedes albopictus) and La Crosse encephalitis (Ae. triseriatus)" (Mol Genet Genomics. 265:66-71 (2001)); Lorenzen MD, Berghammer AJ, Brown SJ, Denell RE, Klinger M, Beeman RW "piggyBac-mediated germ-line transformation in the beetle Tribolium castaneum" (Insect Molec. Biol. 12:433-440 (2003)); Hacker U, Nystedt S, Barmchi MP, Horn C, Wimmer EA "piggyBac-based insertional mutagenesis in the presence of stably integrated P elements in Drosophila" (Proc Natl Acad Sci USA. 100 7720-5 (2003)); Sumitani M, Yamamoto DS, Oishi K, Lee JM, Hatakeyama M. "Germ-line transformation of the sawfly, Athalia rosae (Hymenoptera: Symphyta), mediated by a piggyBac-derived vector" (Insect Biochem. Mol. Biol. 33:449-458 (2003)); Horn C, Offen N, Nystedt S, Haecker U, Wimmer EA. "piggyBac-based insertional mutagensis and enhancer detection as a tool for functional insect genomics" (Genetics. 163:647-61 (2003)); and Tomita M, Munetsuna H, Sato T, Adachi T, Hino R, Hayashi M, Shimizu K, Nakamura N, Tamura T, Yoshizato K. "Transgenic silkworms produce recombinant human type III procollagen in cocoons" (Nat Biotechnol. 21, 52-6 (2003)).

It is further intended in the present disclosure that the transgenic expression can be regulated by an expression control sequence. Thereby, one or more genes to be introduced are placed under the control of (a) suitable expression control sequence(s). Expression can be regulated at the level of the mRNA or polypeptide. It follows therefore, that the term expression control sequence includes mRNA-related elements and protein-related elements. Such elements include promoters, domains within promoters, upstream elements, enhancers, elements that confer tissue or cell specificity, response elements, ribosome binding sequences, transcriptional terminators, etc. An expression control sequence is functionally linked to a nucleotide coding sequence when the expression control sequence is positioned in such a manner to effect or achieve expression of the coding sequence. For example, when a promoter is functionally linked 5' to a coding sequence, expression of the coding sequence is driven by the promoter.

Suitable expression control sequences that can function in silk-producing Lepidopteran cells will be evident to the skilled worker. In some embodiments, it is desirable that the expression control sequence comprises a constitutive promoter. Examples of suitable promoters which can be used include the baculovirus promoters for the p10, polyhedrin (polh), p 6.9, capsid, and cathepsin-like genes. Other suitable strong constitutive promoters include the *B. mori* actin gene promoter; *Drosophilia* hsp70, actin, α-1-tubulin or ubiquitin gene promoters; RSV or MMTV promoters; copia promoter; gypsy promoter; and the cytomegalovirus (CMV) IE gene promoter. Also suitable are the "weaker" baculovirus promoters for the ie1, ie2, ie0, etl, 39K (aka pp31), and gp64 genes. If it is desired that the amount of gene expression from a weak promoter should be increased, enhancer elements, such as the baculovirus enhancer element, hr5, may be used in combination with the promoter. Clearly, the list of possible promoters to be used is not limited to the ones mentioned above. It is evident to the person skilled in the art that the promoter can be chosen according to the respective needs.

The promoter may be a large or complex promoter, but such promoters have the weakness of being inadequately utilised when introduced into non-host Lepidopterans. Accordingly, minimal promoters, such as the hsp70 promoter, can be employed which, while having an intrinsically low level of activity, can be suitably enhanced through a positive feedback mechanism.

A minimal promoter will generally have sufficient additional sequences to permit basal and/or regulated transcription of a gene to be effective. Other sequence information, such as the sequence information which determines tissue specificity, is usually absent. Minimal promoters are, normally as a direct result of this absence, largely inactive without the presence of an active enhancer. Thus, a system of the disclosure will generally require a suitable enhancer or other regulatory element (typically the gene product) to be de-repressed or activated to make the system active when the or each promoter is a minimal promoter.

Thus, it will be appreciated that the minimal promoters may be obtained directly from known sources of promoters, or derived from larger naturally occurring or otherwise known, promoters. Suitable one of the minimal promoters and how to obtain them will be readily apparent to those skilled in the art. For example, suitable ones of the minimal promoters could include ones derived from hsp70, P, and CMV, an Act5C-based minimal promoter, a promoter fragment of BmA3, and an Adh core promoter [Bieschke, E., Wheeler, J., and Tower, J. "Doxycycline-induced transgene expression during Drosophila development and aging" Mol Gen Genet 258, 571-579 (1998)].

Not all of the minimal promoters will necessarily work in all species of insect, but it is readily apparent to those skilled in the art as to how to ensure that the promoter is active. For example, a plasmid, or other vector, comprising a cistron of the disclosure with the minimal promoter to be tested further comprises a marker, such as a gene encoding a fluorescent protein, under the control of a promoter known to work in that species, the method further comprising assaying putative transgenic individuals for expression of the marker, and wherein individuals expressing the marker are then assayed for expression of the gene under the control of the minimal promoter, such as by assaying transcribed RNA. The existence of the RNA above background levels under induced or de-repressed conditions is suggestive that the minimal promoter is active in the particular Lepidopteran species being investigated. Likewise, the presence at low levels only or absence of such RNA in non-induced or repressed conditions is indicative that the minimal promoter may have low intrinsic basal activity.

The expression control sequences to which each recombinant nucleic acid encoding a paralytic peptide is functionally linked may be the same or different. In all of the embodiments discussed herein in which expression control sequences regulate the expression of more than one nucleic acid sequence, the expression control sequences may be the same or different. The integrated copies may be integrated in tandem, integrated into different regions of the same chromosome, or integrated into different chromosomes. The effector gene can be linked with the feedback gene by placing the two genes (effector and feedback gene) in tandem, including the possibility of providing the two genes (effector and feedback gene) as a fusion product, or for example by providing each gene with its own promoter in opposite orientations but in juxtaposition to the enhancer site.

For the purposes of this disclosure the expression control sequence can be regulated (e.g., comprises an inducible promoter and/or enhancer element). Suitable promoters that can be regulated include but are not limited to, e.g. *Drosophila* or other hsp70 promoters, an ecdysone-regulated promoter, the *Saccharomyces cerevisciae* Gal4/UAS system (described elsewhere herein), and other well-known inducible systems. Baculovirus late or very late promoters that are only activated following infection by a baculovirus constitute another type of inducible promoter.

The induction or repression of transgenic expression is termed an "expression switch". The expression switch is simple to control for someone skilled in the art, and is designed such that onset of paralysis will occur shortly after introduction of the inducing factor and in a predictable way, such that a defined number of silk-producing Lepidopterans can be paralysed at any one time.

For example, the expression switch can be controlled through interaction of the silk-producing Lepidopteran larvae with a non-toxic chemical (for example, tetracycline) to form a chemically controlled switch. Tetracycline, for example, can be diluted with water and applied to the silk-producing Lepidopteran larvae via a vapouriser, or administered through ingestion with food in the final meal before spinning. The chemically controlled switch is incorporated in series with the juvenile hormone/ecdysone switch to prevent premature expression of bmPP at any instar by contamination with the chemical and resulting in cessation of feeding and death.

A molecular switch that can be regulated by tetracycline may be used in conjunction with any constitutive promoter, such as those described above (e.g., in conjunction with the CMV-IE promoter, or baculovirus promoters). Thereby, the expression of the gene(s) of interest can be easily controlled by the skilled person.

In general, the product capable of positive transcriptional control may bind to an enhancer located in proximity to the promoter or promoters, thereby serving to enhance polymerase binding at the promoter, for example. Positive transcriptional control may also be attained through other mechanisms, such as mechanisms which counter the effects of a repressor, such as the blocking of an inhibitor of transcription or translation. The use of hairpin RNA's or ribozymes to block translation of the mRNA encoding the transcription inhibitor, for example, may block transcription inhibitors or the product may bind the transcription inhibitor directly, thereby preventing inhibition of transcription or translation.

For example, according to this disclosure the expression of a paralysis-inducing polypeptide can be linked with juvenile hormone (JH). Juvenile hormone is produced in the corpora allata of insects. JH will disperse throughout the hemolymph and act on responsive tissues. JH is principally degraded by the enzymes juvenile hormone esterase (JHE) or juvenile hormone epoxide hydrolase (JHEH). JHE and JHEH both lead to suppression of JH signalling and response. Tissues responsive to JH may produce one or both of these enzymes. As such a transgene could, for example, contain expression control elements that would inhibit the action of these degrading enzymes and hence prolong the effect of the inducible gene, in this case a paralysis-inducing polypeptide.

The mechanism utilised can be, for example, a positive feedback mechanism, wherein the product, which may either be RNA or the translation product thereof (namely, the functional paralysis-inducing polypeptide or protein to be expressed, and at least one promoter therefor), acts at a transcription enhancer site, normally by binding the site, thereby enhancing promoter activity. As one embodiment, the enhancer is associated with the promoter, the gene product serving to enhance activity of the promoter via the enhancer. Enhancement of the promoter activity then increases transcription of the gene for the product which, in turn, further serves to either remove inhibition or enhance promotion, thereby creating a positive feedback loop.

For example, the control factor can be a tTA gene product or an analogue thereof, and one or more tetO operator units is functionally linked with the promoter and is the enhancer, with tTA or its analogue enhancing activity of the promoter via tetO. The gene product of tTA (tetracycline-repressible transcription activator) acts at the tetO operator sequence [Baron U, Bujard H "Tet repressor-based system for regulated gene expression in eukaryotic cells: principles and advances" (Methods Enzymol 327:401-421 (2000)); Gossen M, Freundlieb S, Bender G, Müller G, Hillen W, Bujard H "Transcriptional activation by tetracyclines in mammalian cells" (Science 268:1766-1769 (1995)); Gossen M, Bujard H "Tight control of gene expression in mammalian cells by tetracycline-responsive promoters" (Proc Natl Acad Sci USA 89:5547-5551 (1992))]. Upstream of a promoter, in either orientation, tetO can enhance the levels of transcription from a promoter in close proximity thereto, when bound by the product of the tTA gene. If the tTA gene is part of the construct cassette comprising the tetO operator together with the promoter, then positive feedback will occur when the tTA gene product is expressed.

This system is readily controlled by exposure to tetracycline, which prevents transactivation at tetO by binding to the gene product. Control of the feedback mechanism, in the case of tTA or an analogue thereof, is therefore easily effected by the presence or absence of the tetracycline, or through adjusting the tetracycline concentration, when the tTA gene product is used.

Thereby, tTA serves as a tetracycline-controllable transcription factor. Another example would be the streptogrammin-regulated expression system. It will be appreciated that the transcription factor concerned will determine the binding sites for the appropriate transcription factors.

In an alternative embodiment the expression switch is controlled by temperature (e.g. *Notch*) to form a temperature-controlled switch. One example of a suitable promoter for a temperature-controlled switch is the hsp70 heat shock promoter, allowing the user to control expression by variation of the environmental temperature to which the silk-producing Lepidopterans are exposed in a lab or in the mass-rearing facility, for instance. Another example of the temperature-control is described in Fryxell KJ and Miller TA "Autocidal Biological Control: A General Strategy for Insect Control Based on Genetic Transformation with a Highly Conserved Gene" (Journal of Economic Entomology 88, 1221-1232 (1995)). The temperature at which the expression is switched on can be selected to enable the expression of the selected gene to be upregulated rapidly at a temperature significantly below that at which the silk-producing Lepidopteran larvae are reared (24-30°C). The delay in recovery from, e.g., bmPP or its analogue is such that following paralysis the silk-producing Lepidopteran larvae can be returned to an optimum temperature range for artificial silking without moving until all the silk is reeled. The temperature-controlled switch is incorporated in series with the juvenile hormone/ecdysone switch to prevent incidental chilling sufficient to operate the temperature switch of the silk-producing Lepidopteran larvae, resulting in premature expression of bmPP at any instar. For example, GAL4, further being controllable by use of GAL80 or mutants thereof, may be controlled by temperature, thereby suppressing the effective gene, for example a paralytic polypeptide, until the appropriate stimulus is delivered. In this system, the yeast GAL4 protein is expressed in particular cells by using selected enhancers or promoters. The GAL4 transcription factor activates transcription of reporter genes of choice upon binding to the GAL4 upstream activating sequence (UAS) [Brand AH, Perrimon N "Targeted gene expression as a means of altering cell fates and generating dominant phenotypes" (Development 118, 401-415 (1993))].

In *Drosophila,* ubiquitous expression of GAL80 with a tubulin promoter fully suppresses GAL4-driven pan-neural GFP expression [Lee T, Luo L "Mosaic analysis with a repressible cell marker for studies of gene function in neuronal morphogenesis." (Neuron 22, 451-461 (1999))]. The GAL80 protein antagonizes GAL4 activity by binding to the activation domain of GAL4 through dimer-dimer interactions, preventing interaction between GAL4 and the transcriptional machinery. It is conceivable therefore that the use of GAL80 along with a GAL4/UAS marker would achieve repressible expression of the marker gene of interest.

In another embodiment, expression of bmPP or its analogue is controlled by a chemically repressible switch. Rearing transgenic silk-producing Lepidopteran larvae on a diet containing a non-toxic chemical capable of repressing the expression of, e.g., bmPP through a negative-feedback loop (e.g. tetracycline/tTAV), results in paralysis following the cessation of eating by the final instar silk-producing Lepidopteran larvae. It is necessary to incorporate the chemically repressable switch in series with the juvenile hormone/ecdysone switch to prevent temporary periods of fasting, for example during shedding, resulting in premature expression of bmPP or its analogue.

Accordingly, it will be appreciated that the induced or non-repressed expression level can be modified in a useful and predictable way by adjusting the sequence of the positive feedback system.

Paralysis and/or activity of the paralytic polypeptide can be modified independently of the transcriptional and translational control signals by several approaches, e.g. use of a nuclear localisation signal, modification of the activation domain, etc. [see Fussenegger, M. "The impact of mammalian gene regulation concepts on functional genomic research, metabolic engineering, and advanced gene therapies" Biotechnol Prog 17, 1- 51 (2001)].

In addition, a marker can be included with the transgenic systems of the disclosure. Transformation success rates in the silk-producing Lepidopterans are extremely low, so it is useful to be able to easily identify transgenic silk-producing Lepidopterans in some way. The marker may be introduced by itself, or in conjunction with one or more other heterologous polypeptides. A marker such as this may be used, e.g., to confirm construct functionality, to identify those silk-producing Lepidopteran larvae in which the heterologous construct is being expressed, etc. Suitable markers will be evident to the skilled worker and include but are not limited to no-toxic fluorescent proteins, e.g., DsRed2, green fluorescent protein (GFP), ECFP, and derivatives of EGFP.

Methods for introducing constructs into an embryo to generate a transgenic Lepidopteran (e.g., by microinjection) are conventional. Survivorship varies between Lepidopteran species but is usually quite high (up to 75%) for someone skilled at microinjecting embryos. In general, pre-blastoderm eggs are pierced with a fine glass capillary holding a solution of the plasmid DNA and/or the recombinant virus. G1 Lepidopteran larvae hatched from backcrossing survivors of virus-injected eggs are then screened for expression of the gene of interest. Breeding transgenic G1's with normal Lepidopterans results in Mendelian inheritance. The inventor has succeeded in generating transformants in three species of Lepidoptera *(C. pomonella, P. gossypiella and B. mori*) using a *piggyBac* transposon.

Once the transgene(s) is integrated in a stable manner into the genome of the silk-producing Lepidopteran egg or the early embryos, conventional methods can be used to generate the transgenic silk-producing Lepidoptera, such that the transgene(s) is present in all somatic and germ cells. In another embodiment, when different levels of the paralytic expression are obtained between two or more individual ones of the transgenic silk-producing Lepidopterans, these transgenic silk-producing Lepidopterans can be genetically crossed to produce a transgenic silk-producing Lepidopteran that expresses a greater level, or a complete level, of paralytic expression.

In some embodiments, the transgenic silk-producing Lepidopterans are heterozygous for the paralytic polypeptide genes. For example, when potentially lethal paralytic polypeptides are produced constitutively, it may be advantageous for the silk-producing Lepidopterans to be heterozygous, to limit the amount of the peptide that is produced. In other embodiments, the silk-producing Lepidopterans are homozygous for the transgenes. Methods for producing homozygous transgenic silk-producing Lepidopterans (e.g. using suitable back-crosses) are conventional.

It is accepted that, for different species, different levels of expression are appropriate. Since the sequence-specific transcription factors used to drive the positive feedback system can also be used to express other genes, this bipartite expression system can be achieved by making two separate constructs. A first construct is made with the driver (normally a promoter-transcription factor construct, here the positive feedback construct). A second construct is made with the gene or molecule of interest under the control of a composite promoter (binding site + minimal promoter) responsive to the transcription factor of the first construct [Bello, B., Resendez-Perez, D. and Gehring, W. J. "Spatial and temporal targeting of gene expression in Drosophila by means of a tetracycline-dependent transactivator system" Development, 125,2193 -2202 (1998)]. This is also appropriate for these positive feedback drivers. Alternatively, both constructs with the two functional elements (driver and binding site-minimal promoter-gene of interest) may be combined on the same construct. This combination on the same construct has potentially benefits in a mass rearing setting as the combination very substantially reduces the risk that the two functional elements can be separated by recombination. In addition to this, the complete expression system can be introduced through a single transformation event, as well as meaning that Lepidopterans homozygous for the system are homozygous at only one locus rather than two, which improves the rate of success of construct of the system, by breeding, and reduces the potential fitness cost due to random insertional mutagenesis.

Methods for cloning and expressing polypeptides in silk-producing Lepidopterans are conventional. A sequence "obtained" from a particular source does not necessarily encode a polypeptide sequence identical to that of the wild type paralysis-inducing polypeptide from that source. Any polypeptide that retains the paralytic function of the wild type enzyme, including naturally occurring allelic variants or mutations that are introduced artificially into the protein, can be used. Fragments of the polypeptide that induce paralysis can also be used.

Methods for designing and preparing the constructs suitable for generating transgenic silk-producing Lepidopterans (or vectors for infection of a silk-producing Lepidopteran) are conventional. For these methods, as well as other molecular biology procedures related to the disclosure, see, e.g., Sambrook J and Russell D, "Molecular Cloning: A Laboratory Manual", Third Edition, Cold Spring Harbor, N.Y., (2001); Wu W, Welsh MJ, Kaufman PB, Zhang HH "Methods in Gene Biotechnology", Second edition, CRC Press, New York, N.Y., (2003), "Recombinant Gene Expression Protocols", in Methods in Molecular Biology, Vol. 62, Tuan, ed., Humana Press, Totowa, N.J., (1997); and "Current Protocols in Molecular Biology", Ausabel et al, eds., John Wiley & Sons, N.Y. (1994-2010).

Non-transgenic methods of inducing paralysation in 5^{th} instar silkworm and the subsequent effects on reeling and silk properties can be performed and investigated as depicted in the following example without being limited to this one example:

### Example 1

Bombyx mori silkworms were injected at the base of their right first pseudopod with either 0.1ml of hemolymph that had been extracted and exposed to air for 10 minutes, or with 0.1ml of 100ng/µl paralytic peptide in water solution.

The silkworms injected with the hemolymph were paralysed within 5 minutes, with the exception of some continued movement of the mandibles and thoracic legs of the silkworm. The silkworms injected with the hemolymph remained immobile for several hours, and could be reeled from immediately. Injecting an artificially synthesised version of BmPP also immobilised the worms quickly but the animals often required longer than hemolymph injected worms before giving silk and the effects of the peptide injection lasted for less time than the hemolymph injection.

For both methods it was possible to reel silk samples at a variety of speeds from 10mm/s and 25mm/s. The silkworms injected with hemolymph were simply restrained with a small amount of tape at the hips and the silk was reeled straight onto special analytical spools. The silkworms injected with the synthetic peptide were studied with more advanced restraints to allow for further experimentation into the effects of different post-draw interventions on the properties of the silks.

Silks obtained from the silkworms paralysed with the synthetic peptide all showed less scatter of their stress strain curves compared to silks of unparalysed ones of the silkworms, as well as more consistent cross-sectional areas. This increase of silk quality was significant despite differences in reeling speeds demonstrating that silks from peptide paralysis decrease variation of the fibres consistently. Interestingly, although hemolymph injection induced paralysis resulted in much easier manipulation of and reeling from the paralysed silkworms, the silk samples obtained after hemoplymph injection did not show the significant improvement of consistency in stress strain of samples obtained from larvae paralysed with a synthetic peptide.

Paralysing the silkworm larva enabled experiments that otherwise would not have been possible, such as drawing several hundred meters of silk directly from the silkworm on a reeling spool or reeling silk from the silkworm directly into a post-draw stretching device N and a wash bath L with features such as gum-stripping, water bathing and chemical coating. Such a post-draw stretching device N is depicted in Figure 4.

Figure 4 further shows a paralysed silkworm F, a sensor or set of sensors G for silk dimensions and properties, for example filament motion and tension, possibly containing thermistors for heating and cooling, reeled silk H, post-draw reels I for reeling the reeled silk H, an actuator J for stretching the reeled silk H, an actuator sensor K for sensing the stretching of the reeled silk H, the wash bath L through which the reeled silks H are reeled, and a collecting reel M. Depending on the level of treatment, such post-extrusion modification of the silks significantly affect mechanical properties as well as other tunable properties. By way of example only, Figure 5 demonstrates the effect of one specific treatment (small scale post-draw elongation/stretching by 10 and 16%) on force reeled silk fibre shown here in relation to silk reeled directly from a cocoon. The curves demonstrate stress as a function of strain elongation.

Post-draw stretching within the meaning of the present disclosure means stretching or elongating the silk during and after reeling the silk from the immobilised silk-producing Lepidopteran larvae.

### List of reference numbers

- 200: immobilisation of silk-producing Lepidopteran larvae by paralysis
- 210: transgenic expression of a polypeptide with paralytic effects in the silk-producing Lepidopteran larvae
- 220: injection of hemolymph, wherein the hemolymph is drawn from silk-producing Lepidopteran larvae and oxidised prior to the injection
- 230: injection of a natural or artificial polypeptide with paralytic effects
- 240: direct and artificial silking from the silk-producing Lepidopteran larvae
- 300: mechanical reeling mechanism
- A: reels
- B: motors
- C: Silk-producing Lepidopteran larvae
- D: device to hold many spinning larvae
- E: wash bath of mechanical reeling mechanism 300
- F: paralysed silkworm/immobilised silk-producing Lepidopteran larva
- G: sensors for silk parameters as well as thermistors for heating and cooling
- H: reeled silk
- I: post-draw reels
- J: actuator for stretching the silk
- K: actuator sensor
- L: wash bath of post-draw stretching device N
- M: collecting reel
- N: post-draw stretching device

## Claims

1. A method for immobilising silk-producing Lepidopteran larvae to allow for continuous forced silking comprising:
paralysing the silk-producing Lepidopteran larvae by at least one of
a. transgenically expressing a polypeptide having paralytic effects in the silk-producing Lepidopteran larvae,
b. injecting hemolymph, wherein the hemolymph is drawn from silk-producing Lepidopteran larvae and oxidised prior to the injecting, or
c. injecting a natural or artificial polypeptide having paralytic effects.

2. The method of claim 1, wherein the polypeptide is a protein.

3. The method of any one of the above claims, wherein the transgenically expressing comprises at least one of
a. introducing a viral vector,
b. introducing a transposon-based vector, or
c. regulating by an expression control sequence.

4. The method of claim 3, wherein the expression control sequence comprises at least one of inducible promoter or enhancer element for further controlling the transgenically expressing, or wherein the viral vector or the transposon-based vector further comprises a marker gene.

5. The method of claim 4, wherein the controlling of the transgenically expressing comprises at least one of
a. applying chemicals for inducing transgenic expression,
b. applying temperature for inducing transgenic expression, or
c. applying chemicals for repressing transgenic expression.

6. The method of any one of the above claims, wherein the polypeptide having paralytic effects is *Bombyx mori* paralytic peptide (BmPP).

7. The method of claim 6, wherein the bmPP is mutated but retaining the paralytic effects.

8. The method of any one of the above claims, further comprising:
modifying at least one of paralysis or activity of the polypeptide having paralytic effects independently of transcriptional control signals or translational control signals.

9. Use of the method of one of claims 1 to 8 for immobilising silk-producing Lepidopteran larvae.

10. A method for forced silking from silk-producing Lepidopteran larvae comprising:
immobilising the silk-producing Lepidopteran larvae according to the method of at least one of claims 1 to 8 and directly and artificially silking from the silk-producing Lepidopteran larvae.

11. A device for forced silking from an immobilised silk-producing Lepidopteran larva, comprising
a mechanical reeling mechanism (300), to which are attached the immobilised silk-producing Lepidopteran larvae (C), whereby the immobilised silk-producing Lepidopteran larvae (C) are immobilised according to the method of at least one of claims 1 to 8, and a take up device for the silk.

12. The device of claim 11, being adapted to work at a variety of speeds and onto a variety of reels (A) either singly or multiply in parallel, or wherein brins from the immobilised silk-producing Lepidopteran larva (C) are twisted together during silking with a controlled number of twists per unit length.

13. The device according to either of the claims 11and 12 comprising environmental controllers to control environmental conditions whereby the environmental conditions comprise temperature and humidity.

14. A device (N) for post-draw stretching of silk (H), comprising one or more sensors (G), one or more post-draw reels (I), an actuator (J), an actuator sensor (K), and a collecting reel (M), whereby immobilised silk-producing Lepidopteran larvae (F) are attached to the post-draw stretching device (N) and the immobilised silk-producing Lepidopteran larvae (F) are immobilised according to the method of at least one of claims 1 to 8.

15. The device of claim 14 comprising a wash bath (L) through which the silk (H) is reeled or comprising temperature controllers to adjust temperature of the immobilised silk-producing Lepidopteran larvae (F).

## Patentansprüche

1. Verfahren für das Immobilisieren von Seide-produzierenden Lepidoptera-Larven, um das kontinuierliche Zwangs-Spinnen von Seide zu erlauben, welches umfasst:
Paralysieren der Seide-produzierenden Lepidoptera-Larven durch wenigstens eins von
a. transgen Exprimieren eines Polypeptids mit paralytischen Effekten in den Seide-produzierenden Lepidoptera-Larven,
b. Injizieren von Hämolymphe, wobei die Hämolymphe von Seide-produzierenden Lepidoptera-Larven gewonnen wird und vor dem Injizieren oxidiert wird, oder
c. Injizieren eines natürlichen oder künstlichen Polypeptids mit paralytischen Effekten.

2. Verfahren nach Anspruch 1, wobei das Polypeptid ein Protein ist.

3. Verfahren nach einem der obigen Ansprüche, wobei das transgen Exprimieren wenigstens eins von
a. Einbringen eines viralen Vektors,
b. Einbringen eines Transposon-basierenden Vektors, oder
c. Regulieren durch eine Expressions-Kontrollsequenz umfasst.

4. Verfahren nach Anspruch 3, wobei die Expressions-Kontrollsequenz wenigstens eins eines induzierbaren Promotors oder Enhancer-Elements für das weitere Kontrollieren des transgen Exprimierens umfasst, oder wobei der virale Vektor oder der Transposon-basierende Vektor weiterhin ein Markergen umfasst.

5. Verfahren nach Anspruch 4, wobei das Kontrollieren des transgen Exprimierens wenigstens eins von
a. Applizieren von Chemikalien, um die transgene Expression zu induzieren,
b. Applizieren von Temperatur, um die transgene Expression zu induzieren, oder
c. Applizieren von Chemikalien, um die transgene Expression zu reprimieren, umfasst.

6. Verfahren nach einem der obigen Ansprüche, wobei das Polypeptid mit paralytischen Effekten *Bombyx mori* paralytisches Peptid (BmPP) ist.

7. Verfahren nach Anspruch 6, wobei das bmPP mutiert ist, aber die paralytischen Effekte behält.

8. Verfahren nach einem der obigen Ansprüche, welches weiterhin umfasst:
Modifizieren wenigstens eins der Paralyse oder der Aktivität des Polypeptids mit paralytischen Effekten unabhängig von transkriptionalen Kontrollsignalen oder translationalen Kontrollsignalen.

9. Verwendung des Verfahrens von einem der Ansprüche 1 bis 8 für die Immobilisierung Seide-produzierender Lepidoptera-Larven.

10. Verfahren für das Zwangs-Spinnen von Seide-produzierenden Lepidoptera-Larven, welches umfasst:
Immobilisieren der Seide-produzierenden Lepidoptera-Larven nach dem Verfahren von wenigstens einem der Ansprüche 1 bis 8 und direkt und künstlich Spinnen von Seide von den Seide-produzierenden Lepidoptera-Larven.

11. Vorrichtung für das Zwangs-Spinnen von Seide von einer immobilisierten Seide-produzierenden Lepidoptera-Larve, welche umfasst
einen mechanischen Haspel-Mechanismus (300), an welchem die immobilisierten Seide-produzierenden Lepidoptera-Larven (C) befestigt sind, wobei die immobilisierten Seide-produzierenden Lepidoptera-Larven (C) nach dem Verfahren von wenigstens einem der Ansprüche 1 bis 8 immobilisiert sind, und eine Aufnahmevorrichtung für die Seide.

12. Vorrichtung nach Anspruch 11, welche angepasst ist, bei einer Vielfalt von Geschwindigkeiten und auf einer Vielfalt von Haspeln (A) entweder einzeln oder mehrfach parallel zu arbeiten, oder wobei die Fasern von der immobilisierten Seide-produzierenden Lepidoptera-Larve (C) während des Spinnens der Seide mit einer kontrollierten Anzahl an Drehungen pro Längeneinheit zusammen gedreht sind.

13. Vorrichtung nach einem der Ansprüche 11 und 12, welche Umwelt-Controller umfasst, um Umweltbedingungen zu kontrollieren, wobei die Umweltbedingungen Temperatur und Luftfeuchtigkeit umfassen.

14. Vorrichtung (N) für das Nach-Auszug-Dehnen der Seide (H), welche einen oder mehrere Sensoren (G) umfasst, eine oder mehrere Nach-Auszug-Haspeln (I), einen Antrieb (J), einen Antriebssensor (K) und eine Sammelhaspel (M), wobei die immobilisierten Seide-produzierenden Lepidoptera-Larven (F) an der Nach-Auszug-Dehn-Vorrichtung (N) befestigt sind und die immobilisierten Seide-produzierenden Lepidoptera-Larven (F) nach dem Verfahren von wenigstens einem der Ansprüche 1 bis 8 immobilisiert sind.

15. Vorrichtung nach Anspruch 14, welche ein Waschbad (L) umfasst, durch welches die Seide (H) gehaspelt wird, oder welche Temperatur-Controller umfasst, um die Temperatur der immobilisierten Seide-produzierenden Lepidoptera-Larven (F) anzupassen.

## Revendications

1. Procédé d'immobilisation de larve lépidoptère productrice de soie pour permettre la production forcée continue de soie, comprenant:
paralyser la larve lépidoptère productrice de soie par au moins l'un de :
a) l'expression transgénique d'un polypeptide ayant des effets paralytiques dans la larve lépidoptère productrice de soie,
b) l'injection d'hémolymphe, l'hémolymphe étant récupérée de la larve lépidoptère productrice de soie et oxydée avant l'injection, ou
c) l'injection d'un polypeptide naturel ou artificiel ayant des effets paralytiques.

2. Procédé selon la revendication 1, dans lequel le polypeptide est une protéine.

3. Procédé selon l'une quelconque des revendications précédentes, dans lequel l'expression transgénique comprend au moins l'un de :
a) l'introduction d'un vecteur viral,
b) l'introduction d'un vecteur à base de transposon, ou
c) la régulation d'une séquence de commande d'expression.

4. Procédé selon la revendication 3, dans lequel la séquence de commande d'expression comprend au moins l'un d'un élément amplificateur ou d'un promoteur inductible pour commander en outre l'expression transgénique, ou dans lequel le vecteur viral ou le vecteur à base de transposon comprend en outre un gène marqueur.

5. Procédé selon la revendication 4, dans lequel l'état de commande de l'expression transgénique comprend au moins l'un de :
a) l'application de composés chimiques pour induire l'expression transgénique,
b) l'application de température pour induire l'expression transgénique, ou
c) l'application de composés chimiques pour empêcher l'expression transgénique.

6. Procédé selon l'une quelconque des revendications précédentes, dans lequel le polypeptide ayant des effets paralytiques est peptique paralytique *Bombyx mori* (BmPP).

7. Procédé selon la revendication 6, dans lequel le BmPP est muté mais garde les effets paralytiques.

8. Procédé selon l'une quelconque des revendications précédentes, comprenant en outre :
la modification d'au moins l'activité ou la paralysie du polypeptide ayant des effets paralytiques, de manière indépendante des signaux de commande de transcription ou des signaux de commande de translation.

9. Utilisation du procédé selon l'une quelconque des revendications 1 à 8 pour immobiliser une larve lépidoptère productrice de soie.

10. Procédé pour production de soie forcée d'une larve lépidoptère productrice de soie comprenant :
l'immobilisation de la larve lépidoptère productrice de soie selon le procédé selon l'une quelconque des revendications 1 à 8, et la production de soie directement et artificiellement par la larve lépidoptère productrice de soie.

11. Dispositif pour la production de soie forcée d'une larve lépidoptère productrice de soie immobilisée, comprenant
un mécanisme d'enroulement mécanique (300), auquel les larves lépidoptères (C) productrices de soie immobilisées sont attachées, les larves lépidoptères (C) productrices de soie immobilisées étant immobilisées selon le procédé selon l'une quelconque des revendications 1 à 8, et un dispositif de récupération de la soie.

12. Dispositif selon la revendication 11, adapté pour travailler à une variété de vitesses et sur une variété d'éléments enrouleurs (A) soit simplement soit de manière multiple en parallèle, ou dans lequel des brins provenant de larves lépidoptères (C) productrices de soie immobilisées sont enroulés ensemble pendant la production de soie, avec un nombre commandé de tours par unité de longueur.

13. Dispositif selon l'une quelconque des revendications 11 à 12, comprenant des contrôleurs environnementaux pour commander les conditions environnementales, dans lequel les conditions environnementales comprennent la température et l'humidité.

14. Dispositif (N) pour étirer après extraction de la soie (H) comprenant un ou plusieurs capteurs (G), un ou plusieurs éléments enrouleurs post-extraction (I), un actuateur (J), un capteur d'actuation (K), et un élément enrouleur collecteur (M), dans lequel des larves lépidoptères (F) productrices de soie immobilisées sont attachées aux dispositifs d'étirements post-extraction (N) et les larves lépidoptères (F) productrices de soie immobilisées sont immobilisées selon le procédé d'au moins l'une des revendications 1 à 8.

15. Dispositif selon la revendication 14, comprenant un bain de lavage (L) à travers lequel la soie (H) est enroulée ou comprenant des contrôleurs de température pour ajuster la température des larves lépidoptères (F) productrices de soie immobilisées.
